(19)

Europäisches
Patentamt
European
Patent Office
Office européen
des brevets

(11)  **EP 4 006 047 A1**

(12)  **EUROPEAN PATENT APPLICATION**
published in accordance with Art. 153(4) EPC

(43) Date of publication:
**01.06.2022   Bulletin 2022/22**

(21) Application number: **20846472.7**

(22) Date of filing: **29.07.2020**

(51) International Patent Classification (IPC):
**C07K 1/14** (2006.01)          **C07K 16/00** (2006.01)
**C12P 21/08** (2006.01)

(52) Cooperative Patent Classification (CPC):
**C07K 1/14; C07K 16/00**

(86) International application number:
**PCT/JP2020/029150**

(87) International publication number:
**WO 2021/020474 (04.02.2021 Gazette 2021/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **31.07.2019   JP 2019141768**

(71) Applicant: **Kyowa Kirin Co., Ltd.
Tokyo 100-0004 (JP)**

(72) Inventors:
• **ESHIMA, Yasunari**
  **Tokyo 100-0004 (JP)**
• **ISHIHARA, Takashi**
  **Tokyo 100-0004 (JP)**

(74) Representative: **Hoffmann Eitle
Patent- und Rechtsanwälte PartmbB
Arabellastraße 30
81925 München (DE)**

(54)    **METHOD FOR PURIFYING ANTIBODY USING ADSORBENT**

(57)    The purpose of the present invention is to provide: a method for purifying an antibody in non-adsorption mode using an inorganic compound that contains silicon dioxide and aluminum oxide, as a method for producing an antibody at a lower cost than the prior art; a method for producing an antibody that includes the purification method; and an antibody, etc., produced by the production method. The present invention pertains to a method for purifying an antibody in non-adsorption mode using an inorganic compound that contains silicon dioxide and aluminum oxide, a method for producing an antibody that includes the purification method, and an antibody produced by the production method.

EP 4 006 047 A1

Fig. 1

**Description**

Technical Field

The present invention relates to a method for purifying an antibody and a method for producing an antibody comprising the purification method.

Background Art

With the development of gene recombination technology, pharmaceutical products containing any of various proteins as an active ingredient have come to be supplied. Particularly in recent years, many pharmaceutical products containing an antibody as an active ingredient have been developed and launched. In addition, the efficient and mass production of such a protein has become an increasingly important issue in the biopharmaceutical industry.

Such a protein is generally produced by culturing a recombinant cell into which a vector containing a gene encoding the target protein is inserted. In the culture solution thereof, other than the target protein, impurities such as various types of medium-derived components, host cell-derived components, or protein-derived byproducts are contained, and therefore, it is very difficult and challenging to simultaneously achieve both purification of the target protein by separating impurities to a purity required as a pharmaceutical product, and efficient and mass production of the target protein.

A method for purifying a protein is generally performed by a combination of different modes of chromatography. In the chromatography, the target protein is separated from impurities, for example, based on a difference in molecular charge, hydrophilicity, size, or the like.

In particular, when the target protein is an antibody, many of the purification processes include a capture step using protein A and a subsequent polishing step. The polishing step is performed for the purpose of removing impurities such as a host cell-derived component or an antibody-derived by-product such as an antibody-derived polymer (High Molecular Weight Species; hereinafter also referred to as HMWS) contained in a purification intermediate after the capture step, and leaked protein A. In the polishing step, column chromatography purification such as cation exchange chromatography (CEX), anion exchange chromatography (AEX), hydrophobic interaction chromatography (HIC), or hydroxyapatite chromatography is performed.

Such column chromatography utilizes the fact that the adsorptivity to the column support differs depending on the substance and the chromatography condition. When the target antibody is adsorbed to the column support, the antibody is adsorbed to the column support and then the column is washed to remove a substance that is not adsorbed to the column support, and then the electrical conductivity or pH is changed to elute the antibody. The column chromatography in such a mode is called an adsorption/desorption mode. On the other hand, when the target antibody is not adsorbed to the column support, a substance that is adsorbed to the column support can be removed by allowing a solution containing the antibody and impurities to pass through the column. The column chromatography in such a mode is called a non-adsorption (flow-through) mode. In the non-adsorption mode, purification can be performed with a higher load amount as compared with the adsorption/desorption mode, and also the operation is simple (Non-Patent Document 1). In general, AEX and HIC are performed in a non-adsorption mode. Further, the purification of an antibody by CEX is performed in an adsorption/desorption mode. However, a method in which CEX is performed in a non-adsorption mode by controlling the pH and the electrical conductivity (Patent Document 1).

The type of impurity to be removed by such column chromatography differs depending on the chromatography support. For example, HMWS is removed by CEX or HIC. A host cell-derived impurity is removed by AEX, HIC, or CEX. (Non-Patent Document 1).

Activated clay and aluminum silicate are each an inorganic compound in which aluminum oxide and silicon dioxide are bound at an arbitrary ratio. These are porous and have an excellent adsorption property, and are mainly used as an inexpensive material derived from a natural product for removal of a pigment of a vegetable oil, industrial dehydration, and adsorption of dioxins in an incinerator or the like. Further, a zeolite is a general term for crystalline aluminosilicates, and is characterized by having a uniform pore diameter and having a cation exchange property.

The zeolite has a property of adsorbing a biopolymer such as a protein, and therefore, its use as a chromatography support has been studied. It is known that the pH of a sample containing a biopolymer to be adsorbed affects the adsorption, and the adsorption becomes maximum near the isoelectric point (pI) of the biopolymer (Non-Patent Document 2).

The silicon/aluminum ratio ($Si/Al_2$ ratio) or the three-dimensional structure of a zeolite differs depending on the production method, and as a result, properties such as hydrophobicity and an ion exchange property also differ. It is known that the biopolymer that can be adsorbed differs depending on the zeolite (Non-Patent Document 2).

As an antibody purification method, methods in which IgG is purified from a mixed sample of two types of proteins using zeolite A or zeolite X are known (Non-Patent Documents 3 and 4). These are methods in which an antibody is adsorbed to a zeolite, and impurities are removed, and then the antibody is eluted.

However, a method for purifying an antibody in a non-adsorption mode using a zeolite as a support is not known, and also there are no reported examples of applying a zeolite as a purification substrate in an industrial production step of a recombinant protein drug or an antibody drug.

Prior Art Documents

Patent Documents

Patent Document 1: JP-T-2010-528076

Non-Patent Documents

Non-Patent Document 1: A. A. Shukla et al., Journal of Chromatography B 2007, 848, 28-39
Non-Patent Document 2: M. Matsui et al., Chemistry-European Journal 2001, 7(7), 1555-1560
Non-Patent Document 3: Y. C. Huang et al., Enzyme Microb. Technol. 1995, 17, 564-569
Non-Patent Document 4: Y. C. Yu et al., Biotechnol. Prog. 1998, 14, 332-337

Summary of the Invention

Problems that the Invention is to Solve

An object of the present invention is to provide, as a method for producing an antibody at a lower cost than the prior art, a method for purifying an antibody in a non-adsorption mode using an inorganic compound containing silicon dioxide and aluminum oxide, a method for producing an antibody comprising the purification method, and an antibody produced by the production method, and the like.

Means for Solving the Problems

The present inventors conducted intensive studies in order to solve the above problems, and as a result, they surprisingly found a method for purifying an antibody by separating the antibody from impurities in a non-adsorption mode using an inexpensive inorganic compound containing silicon dioxide and aluminum oxide, and thus completed the present invention.

The present invention relates to the following [1] to [22].

[1] A method for purifying an antibody, comprising separating an antibody from an impurity in a non-adsorption mode using an inorganic compound containing silicon dioxide and aluminum oxide, thereby obtaining an antibody with a reduced impurity content.

[2] The purification method according to [1], wherein the antibody is a genetically recombinant antibody.

[3] The purification method according to [1] or [2], wherein the impurity is at least one selected from a host cell protein, an antibody-derived polymer, an antibody-derived degradation product, and a DNA.

[4] The purification method according to [1] or [2], wherein the impurity is an antibody-derived polymer.

[5] The purification method according to any one of [1] to [4], wherein the inorganic compound containing silicon dioxide and aluminum oxide is activated clay, aluminum silicate, or a zeolite.

[6] The purification method according to any one of [1] to [5], wherein the inorganic compound containing silicon dioxide and aluminum oxide is aluminum silicate.

[7] The purification method according to [6], wherein the aluminum silicate has at least one property selected from the following (i) to (iv):

(i) a specific surface area is 300 to 800 $m^2/g$;
(ii) a pore volume is 0.1 to 0.5 mL/g;
(iii) an average pore diameter is 1.5 to 4 nm; and
(iv) a silica/alumina ratio is 1 to 5.

[8] The purification method according to [6] or [7], wherein the aluminum silicate is Galleon Neutral D2-Y (Mizusawa Industrial Chemicals, Ltd., Galleon is a registered trademark) or Neobead SA (Mizusawa Industrial Chemicals, Ltd.,

Neobead is a registered trademark).

[9] The purification method according to any one of [1] to [5], wherein the inorganic compound containing silicon dioxide and aluminum oxide is activated clay.

[10] The purification method according to [9], wherein the activated clay has at least one property selected from the following (i) to (iv):

(i) a specific surface area is 100 to 400 m$^2$/g;
(ii) a pore volume is 0.2 to 0.6 mL/g;
(iii) an average pore diameter is 3 to 10 nm; and
(iv) a silica/alumina ratio is 6 to 10.

[11] The purification method according to [9] or [10], wherein the aluminum silicate is Galleon Earth NVZ, Galleon Earth NV, Galleon Earth V2R, or Galleon Earth V2 (Mizusawa Industrial Chemicals, Ltd., Galleon is a registered trademark).

[12] The purification method according to any one of [1] to [5], wherein the inorganic compound containing silicon dioxide and aluminum oxide is a zeolite.

[13] The purification method according to [12], wherein the zeolite has at least one property selected from the following (i) to (iii):

(i) a specific surface area is 100 to 800 m$^2$/g;
(ii) a pore diameter is 0.2 to 1 nm; and
(iii) a silica/alumina ratio is 2 to 1500.

[14] The purification method according to [12] or [13], wherein the zeolite is a β-type or mordenite-type zeolite.

[15] The purification method according to any one of [12] to [14], wherein the zeolite contains a proton as a counter cation.

[16] The purification method according to any one of [1] to [15], wherein the separation of the antibody from the impurity is performed at pH 4 to 9.

[17] The purification method according to any one of [1] to [15], wherein the separation of the antibody from the impurity is performed at pH 6 to 9.

[18] The purification method according to any one of [1] to [15], wherein the separation of the antibody from the impurity is performed at pH 7.5 to 8.5.

[19] The purification method according to any one of [1] to [18], which comprises equilibrating the inorganic compound containing silicon dioxide and aluminum oxide with a buffer solution as a pretreatment.

[20] A method for producing an antibody, comprising the purification method according to any one of [1] to [19].

[21] The production method according to [20], which comprises at least one of protein A chromatography, anion exchange chromatography, cation exchange chromatography, hydrophobic interaction chromatography, mixed mode chromatography, and activated carbon chromatography.

[22] An antibody produced by the production method according to [20] or [21].

Effects of the Invention

According to the present invention, as a method for producing an antibody at a lower cost than the prior art, a method for purifying an antibody in a non-adsorption mode using an inorganic compound containing silicon dioxide and aluminum oxide, a method for producing an antibody comprising the purification method, and an antibody produced by the production method, and the like are provided.

Brief Description of the Drawings

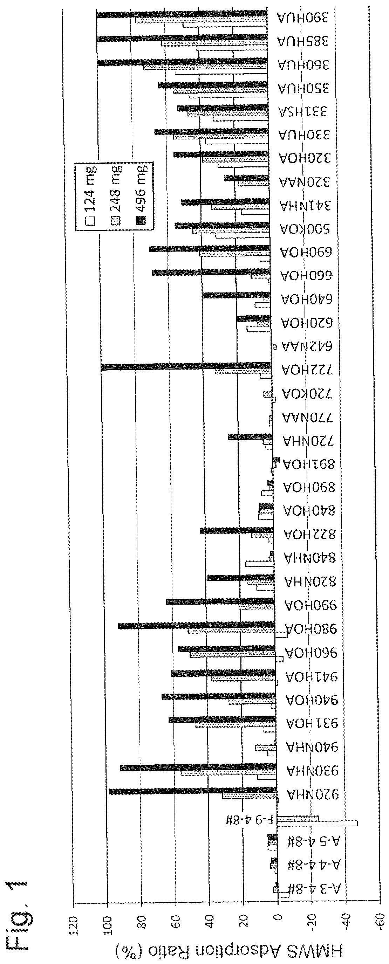

[Fig. 1] Fig. 1 shows an HMWS adsorption ratio after a culture supernatant of MabA was treated with any of various types of zeolites. The vertical axis represents the HMWS adsorption ratio (%). The white, grey, and black graphs show the results when the zeolite was added in an amount of 124 mg, 248 mg, and 496 mg, respectively.

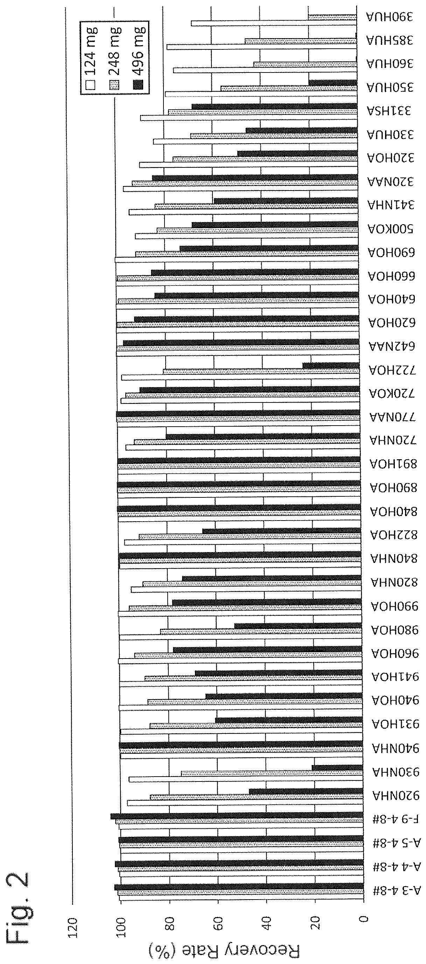

[Fig. 2] Fig. 2 shows a recovery rate after a culture supernatant of MabA was treated with any of various types of zeolites. The vertical axis represents the recovery rate (%). The white, grey, and black graphs show the results when the zeolite was added in an amount of 124 mg, 248 mg, and 496 mg, respectively.

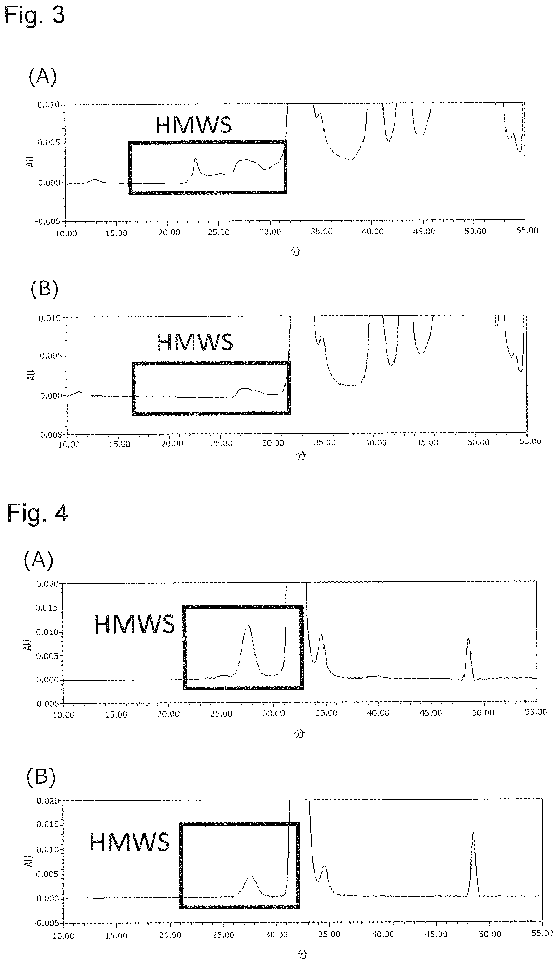

[Fig. 3] Figs. 3(A) and 3(B) show elution patterns obtained by analyzing a culture supernatant of MabA before and after a treatment with a zeolite (HSZ-660HOA) (Tosoh Corporation, HSZ is a registered trademark) through size exclusion chromatography. Fig. 3(A) shows the elution pattern before the treatment with the adsorbent, and Fig. 3(B) shows the elution pattern after the treatment with the adsorbent.

[Fig. 4] Figs. 4(A) and 4(B) show elution patterns obtained by analyzing a crudely purified sample of MabD before and after a treatment with a zeolite (HSZ-660HOA) (Tosoh Corporation, HSZ is a registered trademark) through size exclusion chromatography. Fig. 4(A) shows the elution pattern before the treatment with the adsorbent, and Fig. 4(B) shows the elution pattern after the treatment with the adsorbent.

Mode for Carrying Out the Invention

The present invention relates to a method for purifying an antibody comprising separating an antibody from an impurity in a non-adsorption mode using an inorganic compound containing silicon dioxide and aluminum oxide, thereby obtaining an antibody with a reduced impurity content.

Examples of the antibody include a mouse antibody, a llama antibody, a chimeric antibody, a humanized antibody, a human antibody, an antibody obtained by modifying the Fc region thereof, and the like. Examples of the molecular type of the antibody include IgG, IgM, IgA, IgD, IgE, Fab, Fc, an Fc-fusion protein, VH, VL, VHH, Fab'$_2$, scFv, scFab, scDb, scDbFc, and the like.

In the purification method of the present invention, an antibody-containing aqueous solution containing the target antibody and an impurity is provided.

As the antibody-containing aqueous solution, for example, a composition obtained from a living body such as plasma, serum, milk, or urine, a culture solution of a cell or a bacterium such as E. coli that produces an antibody obtained by using a gene recombination technique or a cell fusion technique, a composition obtained from a transgenic non-human animal, plant, insect, or the like, a composition obtained using a cell-free protein synthesis technique, or the like is exemplified.

As the cell that produces an antibody, for example, a transformed cell in which a gene encoding a desired antibody is incorporated into a host cell, or the like is exemplified.

As the host cell, for example, a cell line such as an animal cell, a plant cell, or a yeast cell is exemplified.

Specifically, for example, Chinese hamster ovary cells (CHO cells), NS0 cells, SP2/0 cells, which are mouse myeloma cells, YB2/0 cells, IR983F cells, which are rat myeloma cells, BHK cells which are syrian hamster kidney-derived cells, namalva cells which are human myeloma cells, embryonic stem cells, fertilized egg cells, and the like are exemplified.

As the culture medium for culturing the cell that produces an antibody, any culture medium is used as long as it is a culture medium suitable for culturing each cell, but for example, as the culture medium for culturing an animal cell, a common culture medium to be used for culturing an animal cell is used. For example, any culture medium such as a serum-containing culture medium, a culture medium containing no animal-derived components such as serum albumin or a serum fraction, a serum-free culture medium, or a protein-free culture medium is used, but preferably, a serum-free culture medium or a protein-free culture medium is used.

Specifically, for example, RPMI 1640 medium [The Journal of the American Medical Association, 199, 519 (1967)], Eagle's MEM medium [Science, 122, 501 (1952)], Dulbecco's modified MEM (DMEM) medium [Virology, 8, 396 (1959)], Medium 199 [Proceeding of the Society for the Biological Medicine, 73, 1 (1950)], F12 medium [Proc. Natl. Acad. Sci. USA, 53, 288 (1965)], Iscove's modified Dulbecco's medium (IMDM medium) [J. Experimental Medicine, 147, 923 (1978)], EX-CELL 302 medium, EX-CELL-CD-CHO medium, EX-CELL 325 medium (which are manufactured by SAFC Biosciences, Inc.), CD-CHO medium, CD DG44 medium (which are manufactured by Invitrogen, Inc.) or IS CD-CHO medium (manufactured by Irvine Scientific Sales Company), or a modified culture medium, a mixed culture medium, or a concentrated culture medium thereof, or the like is used. Among these, RPMI 1640 medium, DMEM medium, F12 medium, IMDM medium, EX-CELL 302 medium, CD-CHO medium, IS CD-CHO medium, or the like is preferably exemplified.

In addition, a biologically active substance, a nutritional factor, or the like necessary for the growth of the cell that produces an antibody can be added as needed. Such an additive is incorporated in the culture medium in advance before culturing, or is additionally supplied to the culture solution as an additional culture medium or an additional solution during culturing as appropriate. The additional supply method may be in any form such as one solution or a mixed solution of two or more types, and the addition method may be either continuous or intermittent.

As the transgenic non-human animal, plant, or insect that produces an antibody, for example, a non-human animal, a plant, or an insect in which a gene encoding an antibody is incorporated into a cell is exemplified. Examples of the non-human animal include mice, rats, guinea pigs, hamsters, rabbits, dogs, sheep, pigs, goats, cattle, monkeys, and the like. Examples of the plant include tobacco, potatoes, tomatoes, carrots, soybeans, rape, alfalfa, rice, wheat, barley, corn, and the like.

As a method for producing the antibody-containing aqueous solution, for example, a method described in WO 2007/062245, WO 2007/114496, or the like is exemplified.

Further, in the present invention, as the antibody-containing aqueous solution, for example, other than those obtained from a living body such as plasma or urine containing an antibody, an antibody-containing aqueous solution obtained in a purification step is exemplified. Specifically, for example, a cell-free liquid, a precipitate-free liquid, an alcohol fractionated liquid, a salting out fractionated liquid, a chromatography eluate, and the like are exemplified.

As the cell-free liquid, for example, a solution obtained by removing cells from an antibody-containing aqueous solution obtained from a living body such as plasma, serum, milk, or urine, an antibody-containing aqueous solution obtained from a transgenic non-human animal, plant, or insect, an antibody-containing aqueous solution obtained from a cell established using a gene recombination technique, or an antibody-containing aqueous solution obtained in a purification step, or the like is exemplified. Specifically, for example, a solution obtained by removing cells from a cell culture solution by a centrifugal separation method, a cross-flow filtration method (tangential flow filtration method), a filtration method using a depth filter, a filtration method using a membrane filter, a dialysis method, or a method in which these methods are combined, or the like is exemplified.

As the precipitate-free liquid, for example, a solution obtained by performing a low pH treatment or coagulation precipitation (flocculation) by adding capric acid, an organic solvent, polyethylene glycol, a surfactant, a salt, an amino acid, a polymer, or the like, or two-phase separation, and thereafter removing a precipitate from an antibody-containing aqueous solution obtained from a living body such as plasma, serum, milk, or urine, an antibody-containing aqueous solution obtained from a transgenic non-human animal, plant, or insect, an antibody-containing aqueous solution obtained from a cell established using a gene recombination technique, an antibody-containing aqueous solution obtained using a cell-free protein synthesis technique, or an antibody-containing aqueous solution obtained in a purification step, or the like is exemplified. As a method for removing a precipitate, for example, a centrifugal separation method, a cross-flow filtration method (tangential flow filtration method), a filtration method using a depth filter, a filtration method using a membrane filter, a dialysis method, or a method in which these methods are combined, or the like is exemplified.

As the alcohol fractionated liquid, for example, a fractionated liquid prepared by adding an alcohol or the like from an antibody-containing aqueous solution obtained from a living body such as plasma, serum, milk, or urine, an antibody-containing aqueous solution obtained from a transgenic non-human animal, plant, or insect, an antibody-containing aqueous solution obtained from a cell established using a gene recombination technique, an antibody-containing aqueous solution obtained using a cell-free protein synthesis technique, or an antibody-containing aqueous solution obtained in a purification step, or the like is exemplified. Specifically, for example, a fractionated liquid obtained by a method such as a cold ethanol fractionation method is exemplified.

As the salting out fractionated liquid, for example, a fractionated liquid prepared by adding a salt such as ammonium sulfate, sodium sulfate, sodium citrate, sodium chloride, or potassium chloride to deposit an antibody from an antibody-containing aqueous solution obtained from a living body such as plasma, serum, milk, or urine, an antibody-containing aqueous solution obtained from a transgenic non-human animal, plant, or insect, an antibody-containing aqueous solution obtained from a cell established using a gene recombination technique, an antibody-containing aqueous solution obtained using a cell-free protein synthesis technique, or an antibody-containing aqueous solution obtained in a purification step, or the like is exemplified.

As the chromatography eluate, for example, an antibody eluate obtained by adsorbing an antibody-containing aqueous solution obtained from a living body such as plasma, serum, milk, or urine, an antibody-containing aqueous solution obtained from a transgenic non-human animal, plant, or insect, an antibody-containing aqueous solution obtained from a cell established using a gene recombination technique, an antibody-containing aqueous solution obtained using a cell-free protein synthesis technique, or an antibody-containing aqueous solution obtained in a purification step to a support or a membrane to be used in chromatography, and then performing elution with an appropriate eluent or allowing such an antibody-containing aqueous solution to pass through a column or a membrane in which a support or the like is enclosed in a non-adsorption mode, or the like is exemplified.

Examples of the support or the membrane to be used in chromatography include an affinity support, an ion exchange support, an ion exchange membrane, a gel filtration support, a hydrophobic interaction support, a reverse phase support, a hydroxyapatite support, a fluoroapatite support, a sulfated cellulose support, a sulfated agarose support, a mixed mode (multimodal) support, an activated carbon, and the like.

As the ion exchange support or ion exchange membrane, a support or a membrane, in which a molecule having an ion exchange group, for example, a sulfate group, a methyl sulfate group, a sulfophenyl group, a sulfopropyl group, a carboxymethyl group, a quaternary ammonium group, a quaternary aminoethyl group, a diethylaminoethyl group, or the like is directly or indirectly bound to a base support or membrane, for example, a polymer such as cellulose, sepharose, agarose, chitosan, an acrylic acid polymer, or a styrene-divinyl benzene copolymer, or a derivative thereof (including a crosslinked polymer), a polymer constituted by silica particles, glass particles, ceramic particles, or surface-treated particles thereof, or the like is exemplified. Specific examples thereof include Q Sepharose XL, Q Sepharose FF, DEAE Sepharose FF, ANX Sepharose FF, Capto Q, Capto DEAE, Capto Q ImpRes (which are manufactured by GE Healthcare, Inc., Capto is a registered trademark), TOYOPEARL GigaCap Q-650, TOYOPEARL SuperQ-650 (which are manufactured by Tosoh Corporation, TOYOPEARL is a registered trademark), Fractogel DEAE, Fractogel TMAE, Fractogel TMAE Hicap, Eshmuno Q (which are manufactured by Merck Millipore Ltd.), Cellufine MAX-Q (manufactured by JNC Corporation), Mustang Q (manufactured by Pall Corporation), Sartobind Q, Sartobind STIC (which are manufactured by Sartorius Corporation), SP Sepharose FF, CM Sepharose FF, SP Sepharose XL, Capto S (which are manufactured by GE Healthcare, Inc.), Poros 50 HS, Poros 50 XS (which are manufactured by Applied Biosystems, Inc.), Eshmuno S, Fractogel COO-, Fractogel SO3-, Fractogel SE Hicap (which are manufactured by Merck Millipore Ltd.), TOYOPEARL GigaCap S-650, TOYOPEARL GigaCap CM-650 (which are manufactured by Tosoh Corporation, TOYOPEARL is a registered trademark), Cellufine MAX-S (manufactured by JNC Corporation, Cellufine is a registered trademark), Mustang S (manufactured by Pall Corporation), Sartobind S (manufactured by Sartorius Corporation), DIAION PK, DIAION PA, DIAION CR, DIAION AMP (which are manufactured by Mitsubishi Chemical Corporation, DIAION is a registered trademark), and the like, but are not limited thereto.

As the affinity support, a support in which a molecule having affinity for the target protein, for example, protein A, protein G, protein L, or the like is directly or indirectly bound to a base support similar to the above is exemplified. Specific examples thereof include MabSelect, Protein A Sepharose FF, MabSelect Xtra, MabSelect SuRe, MabSelect SuRe LX, Protein G Sepharose FF, Capto L (which are manufactured by GE Healthcare, Inc., MabSelect is a registered trademark), Prosep vA Hicapacity, Prosep vA Ultra, Prosep Ultraplus (which are manufactured by Merck Millipore Ltd., Prosep is a registered trademark), and the like, but are not limited thereto.

As the gel filtration support, for example, a support composed of a polymer constituted by dextran, allyl dextran, N,N'-methylenebisacrylamide, cellulose, agarose, styrene, divinylbenzene, polyvinyl alcohol, silica, chitosan, or the like is exemplified. Specific examples thereof include Sephacryl (registered trademark) S series, Sepharose (registered trademark) series, Sephadex (registered trademark) series, Superdex (registered trademark) series, Sephacryl (registered trademark) series (which are manufactured by GE Healthcare., Inc.), TOYOPEARL (registered trademark) HW series, TSKgel (registered trademark) PW series (which are manufactured by TOSOH Corporation), Bio gel Agarose, Bio gel P Polyacrylamide (which are manufactured by Bio-Rad Inc., Bio gel is a registered trademark), Cellufine GH, Cellufine GCL (which are manufactured by JNC Corporation, Cellufine is a registered trademark), Trisacryl GF05, Trisacryl GF2000, Ultrogel AcA (which are manufactured by Pall Corporation, Trisacryl is a registered trademark), Fractogel BioSEC (manufactured by Merck Millipore Ltd.), and the like, but are not limited thereto.

As the hydrophobic interaction support, a support in which a hydrophobic molecule, for example, a methyl group, an ethyl group, a propyl group, an isopropyl group, a butyl group, a tert-butyl group, an octyl group, an ether group, a phenyl group, or the like is directly or indirectly bound to a base support similar to the above is exemplified. Specific examples thereof include Phenyl Sepharose 6 Fast Flow (high-sub), Phenyl Sepharose 6 Fast Flow (low-sub), Octyl Sepharose 4 Fast Flow, Butyl Sepharose 4 Fast Flow (which are manufactured by GE Healthcare, Inc.), TOYOPEARL Hexyl-650, TOYOPEARL Butyl-650, TOYOPEARL Phenyl-650, TOYOPEARL Ether-650, TOYOPEARL PPG-600, TOYOPEARL Butyl-600, TOYOPEARL Super Butyl-550 (which are manufactured by TOSOH Corporation, TOYOPEARL is a registered trademark), Mactro-Prep t-Butyl, Macro-Prep Methyl (which are manufactured by Bio-Rad Inc.), QMA Spherosil, Methyl Ceramic HyperD (which are manufactured by Pall Corporation), Fractogel Phenyl (S), Fractogel Propyl (S) (which are manufactured by Merck Millipore Ltd.), phenyl-Cellulofine (manufactured by JNC Corporation), DIAION HP, DIAION SP (which are manufactured by Mitsubishi Chemical Corporation, DIAION is a registered trademark), butylated Chitopearl, phenylated Chitopearl (which are manufactured by FUJIBO Holdings, Inc., Chitopearl is a registered trademark), and the like, but are not limited thereto.

As the reverse phase support, for example, a support in which a hydrocarbon group is directly or indirectly bound to a solid phase matrix is exemplified. Examples of the hydrocarbon group include a trimethyl group, a butyl group, a phenyl group, an octyl group, an octadecyl group, a functional group with a terminal modification thereof, and the like. Specific examples thereof include RESOURCE RPC series, SOURCE RPC series (which are manufactured by GE Healthcare, Inc., RESOURCE is a registered trademark), and the like, but are not limited thereto.

As the hydroxyapatite support, for example, CHT Ceramic Hydroxyapatite Type I or Type II (which is manufactured by Bio-Rad, Inc.) is exemplified, but it is not limited thereto. In addition, as the fluoroapatite support, for example, CFT Ceramic Fluoroapatite (manufactured by Bio-Rad Inc.), or the like is exemplified, but it is not limited thereto.

As the mixed mode support, a support in which two or more types of functional groups having different selectivity, preferably an ion exchange group similar to the above and a hydrophobic interaction group similar to the above are directly or indirectly bound to a base support similar to the above is exemplified. Specific examples thereof include Capto adhere, Capto MMC (which are manufactured by GE Healthcare, Inc.), HEA HyperCel, PPA HyperCel, MEP HyperCel (which are manufactured by Paul Corporation), TOYOPEARL MX-Trp-650M (manufactured by TOSOH Corporation, TOYOPEARL is a registered trademark), and the like, but are not limited thereto.

Examples of the activated carbon include Carboraffin, strong SHIRASAGI, purified SHIRASAGI, special SHIRASAGI, SHIRASAGI A, SHIRASAGI C, SHIRASAGI C-1, SHIRASAGI DO-2, SHIRASAGI DO-5, SHIRASAGI DO-11, SHIRASAGI DC, SHIRASAGI DO, SHIRASAGI Gx, SHIRASAGI G, SHIRASAGI GH, SHIRASAGI FAC-10, SHIRASAGI M, SHIRASAGI P, SHIRASAGI PHC, SHIRASAGI Gc, SHIRASAGI GH, SHIRASAGI GM, SHIRASAGI GS, SHIRASAGI GT, SHIRASAGI GAA, SHIRASAGI GOC, SHIRASAGI GOX, SHIRASAGI APRC, SHIRASAGI TAC, SHIRASAGI MAC, SHIRASAGI XRC, SHIRASAGI NCC, SHIRASAGI SRCX, SHIRASAGI Wc, SHIRASAGI LGK, SHIRASAGI KL, SHIRASAGI WH, SHIRASAGI W, SHIRASAGI WHA, SHIRASAGI LH, SHIRASAGI KL, SHIRASAGI LGK, SHIRASAGI MAC-W, SHIRASAGI S, SHIRASAGI Sx, SHIRASAGI X2M, SHIRASAGI X7000, SHIRASAGI X7100, SHIRASAGI DX7-3, MOLSIEVON (which are manufactured by Japan EnviroChemicals, Limited, SHIRASAGI is a registered trademark), ACF, Taiko (which are manufactured by Fuji Chemical Corporation, Taiko is a registered trademark), GLC (which is manufactured by KURARAY CHEMICAL Co., Ltd.), Taiko S, Taiko K, Taiko Q (which are manufactured by FUTAMURA Chemical Co., Ltd., Taiko is a registered trademark), GAC, CN, CG, CAP/CGP, SX, CA (which are manufactured by Norit Japan Co., Ltd.), and the like, but are not limited thereto.

Further, when insoluble materials such as particles are present in the antibody-containing aqueous solution, such materials are removed in advance, and the antibody-containing aqueous solution after removing the insoluble materials may be subjected to the purification method of the present invention. As a method for removing insoluble materials such as particles, for example, a centrifugal separation method, a cross-flow filtration method (tangential flow filtration method), a filtration method using a depth filter, a filtration method using a membrane filter, a dialysis method, or a method in which these methods are combined is exemplified. Further, the antibody-containing aqueous solution is subjected to the purification method of the present invention after adjusting the pH, the electrical conductivity, the buffer solution, or the antibody concentration of the below-mentioned antibody-containing aqueous solution, or the antibody loading amount per unit volume or unit weight of the inorganic compound containing silicon dioxide and aluminum oxide, or the like as needed.

As a method for adjusting the pH, the electrical conductivity, the buffer solution, or the antibody concentration, or the antibody loading amount per unit volume or unit weight of the inorganic compound containing silicon dioxide and aluminum oxide, or the like, for example, an ultrafiltration method using an ultrafiltration membrane or the like is exemplified.

As the ultrafiltration membrane, a positively or negatively charged ultrafiltration membrane is also included in addition to a conventional ultrafiltration membrane. Specific examples thereof include Pellicon 3 Ultracel membrane, Pellicon 3 biomax membrane, Pellicon 2 Ultracel membrane, Pellicon 2 biomax membrane (which are manufactured by Merck Millipore Ltd.), Omega membrane (manufactured by Pall Corporation), Kvick membrane (manufactured by GE Healthcare, Inc.), and the like, but are not limited thereto.

In the present invention, as the impurity, a host cell protein (HCP), an antibody-derived polymer (HMWS), an antibody-derived degradation product, an antibody-derived modification product resulting from denaturation, removal of a sugar chain component, oxidation, deamidation, or the like, a DNA, a culture medium-derived component, a culture additive, an enzyme eluted from a host cell, protein A to be mixed in a capture step, or the like is exemplified, and preferably, a host cell protein, an antibody-derived polymer, an antibody-derived degradation product, or a DNA is exemplified.

In the present invention, the antibody-derived polymer includes any polymer as long as it is formed by association or aggregation of multiple antibody molecules, but is preferably a soluble polymer. Specifically, a dimer, a trimer, a tetramer, or a polymer formed by association or aggregation of 5 or more antibody molecules is exemplified, but it is not limited thereto.

In the present invention, as the antibody-derived degradation product, for example, one in which a disulfide bond or a peptide bond of an antibody is cleaved is exemplified. Specifically, one in which the heavy chain or the light chain of a monoclonal antibody is partially or entirely missing is exemplified. In addition, a Fab fragment, one in which the heavy chain and the light chain of a Fab fragment are further cleaved, and the like are also included.

Examples of the enzyme eluted from a host cell include a glycolytic enzyme, a proteolytic enzyme, a redox enzyme, and the like.

Specific examples of the glycolytic enzyme include neuraminidase (sialidase), galactosidase, glycanase, and the like. Specific examples of the proteolytic enzyme include serine protease, esterase, cysteine protease, trypsin-like protease, aminopeptidase, aspartic protease, cathepsin, and the like. Specific examples of the redox enzyme include a thioredoxin-related enzyme such as thioredoxin reductase, and the like. Specific examples of an amino acid isomerase include transglutaminase and the like.

As the inorganic compound containing silicon dioxide and aluminum oxide used in the purification method of the present invention, any compound may be used as long as it is an inorganic compound containing silicon dioxide and aluminum oxide suitable for the production of a pharmaceutical product, and one type of inorganic compound containing silicon dioxide and aluminum oxide may be used alone, or two or more types of inorganic compounds containing silicon dioxide and aluminum oxide may be used after mixing or without mixing.

As the inorganic compound containing silicon dioxide and aluminum oxide, for example, a zeolite, activated clay, aluminum silicate, acidic clay, kaolin, or bentonite is exemplified, preferably a zeolite, activated clay, or aluminum silicate is exemplified, and particularly preferably, a zeolite or aluminum silicate is exemplified, and most preferably a zeolite is exemplified.

As the zeolite used in the present invention, zeolites having any structure are included, and for example, an A-type, β-type, ZSM-5-type, ferrierite-type, mordenite-type, L-type, or Y-type zeolite is exemplified. As the zeolite used in the present invention, a mordenite-type or β-type zeolite is preferred, and a mordenite-type zeolite is more preferred.

Further, the zeolite used in the present invention contains a template or counter cation in the crystal structure. The template is also referred to as a structure directing agent. As the counter cation, any counter cation is included as long as it is a positively charged atom or atomic group, and examples thereof include a proton, an ammonium ion, a sodium ion, and a potassium ion. As the counter cation contained in the zeolite used in the present invention, a proton is preferred.

The specific surface area of the zeolite used in the present invention is preferably 100 to 800 $m^2$/g, more preferably 150 to 700 $m^2$/g, further more preferably 350 to 600 $m^2$/g, and most preferably 350 to 500 $m^2$/g. The pore diameter of the zeolite used in the present invention is preferably 0.2 to 1 nm, more preferably 0.5 to 0.8 nm, further more preferably 0.6 to 0.75 nm, and most preferably 0.65 to 0.75 nm. The silica/alumina ratio of the zeolite used in the present invention is preferably 2 to 1500, more preferably 10 to 1500, and further more preferably 15 to 300.

As the zeolite used in the present invention, specifically, for example, Zeolam A-3, Zeolam A-4, Zeolam A-5, Zeolam F-9, Zeolam NSA-700, HSZ-920NHA, HSZ-930NHA, HSZ-940NHA, HSZ-931HOA, HSZ-940HOA, HSZ-941HOA, HSZ-960HOA, HSZ-980HOA, HSZ-990HOA, HSZ-820NHA, HSZ-840NHA, HSZ-822HOA, HSZ-840HOA, HSZ-890HOA, HSZ-891HOA, HSZ-720NHA, HSZ-770NAA, HSZ-720KOA, HSZ-722HOA, HSZ-642NAA, HSZ-620HOA, HSZ-640HOA, HSZ-660HOA, HSZ-690HOA, HSZ-500KOA, HSZ-341NHA, HSZ-320NAA, HSZ-320HOA, HSZ-330HUA, HSZ-331HSA, HSZ-350HUA, HSZ-360HUA, HSZ-385HUA, HSZ-390HUA (which are from Tosoh Corporation, Zeolam and HSZ are registered trademarks), Zeeklite SGW, Zeeklite SGW-B (which are from Zeeklite Corporation), or the like is exemplified, but it is not limited thereto.

The specific surface area of the activated clay used in the present invention is preferably 100 to 400 $m^2$/g, more preferably 200 to 350 $m^2$/g, and further more preferably 240 to 310 $m^2$/g. The pore volume of the activated clay used in the present invention is preferably 0.2 to 0.6 mL/g, and more preferably 0.3 to 0.5 mL/g. The average pore diameter of the activated clay used in the present invention is preferably 3 to 10 nm, and more preferably 5 to 7 nm. The silica/alumina ratio of the activated clay used in the present invention is preferably 6 to 10.

As the activated clay used in the present invention, specifically, for example, Galleon Earth NVZ, Galleon Earth NV, Galleon Earth V2R, Galleon Earth V2 (which are from Mizusawa Industrial Chemicals, Ltd., Galleon is a registered trademark), activated clay SA35, activated clay SA1, activated clay R-15 (which are from Toshin Chemicals Co., Ltd.), ACTAL20X, ACTAL20, ACTAL10 (which are from Nikki Universal Co., Ltd.), or the like is exemplified, but it is not limited thereto.

The specific surface area of the aluminum silicate used in the present invention is preferably 300 to 800 $m^2$/g, and more preferably 500 to 600 $m^2$/g. The pore volume of the aluminum silicate used in the present invention is preferably 0.1 to 0.5 mL/g, and more preferably 0.25 to 0.4 mL/g. The average pore diameter of the aluminum silicate used in the present invention is preferably 1.5 to 4 nm, and more preferably 2 to 3 nm. The silica/alumina ratio of the aluminum silicate used in the present invention is preferably 1 to 5, and more preferably 3.5 to 4.5.

As the aluminum silicate used in the present invention, specifically, for example, Galleon Neutral D2-Y, Neobead SA (which are from Mizusawa Industrial Chemicals, Ltd., Galleon and Neobead are registered trademarks), or the like is exemplified, but it is not limited thereto.

As the acidic clay used in the present invention, specifically, for example, Mizuka Ace #20, Mizuka Ace #200, Mizuka Ace #600, Mizulite (which are from Mizusawa Industrial Chemicals, Ltd., Mizuka Ace is a registered trademark), or the like is exemplified, but it is not limited thereto.

As the kaolin used in the present invention, specifically, for example, Hydrite TS90, Hydrite UF90, Kaopaque 10 (which are from Hayashi Kasei Co., Ltd.), RC-1, Glomax LL (which are from Takehara Kagaku Kogyo Co., Ltd.), or the like is exemplified, but it is not limited thereto.

As the bentonite used in the present invention, specifically, for example, Kunipia-F, Kunipia-G, Moistonite-U, Moistonite-S, Moistnite-HC (which are from Kunimine Industries, Co., Ltd., Kunipia and Moistonite are registered trademarks), or the like is exemplified, but it is not limited thereto.

A means for the purification method of the present invention is not particularly limited, but examples thereof include a batch method, a membrane treatment method, a column chromatography method, and the like, and an inorganic compound containing silicon dioxide and aluminum oxide of an appropriate type and/or having an appropriate shape according to each means is selected. It can also be used in the form of particles or the like in which the inorganic compound containing silicon dioxide and aluminum oxide is enclosed in a porous polymer or gel, or in the form of a film formed by adsorbing, fixing, or molding the inorganic compound containing silicon dioxide and aluminum oxide using a support agent or a fiber or the like of polypropylene, cellulose, or the like, or in the form of a cartridge or the like in which a powder is sandwiched between films as needed.

Further, it is also possible to appropriately select the crystal structure, the specific surface area, the pore diameter, the silica/alumina ratio, and/or the pH or the like of the inorganic compound containing silicon dioxide and aluminum oxide to be used according to the target antibody and the means of the purification method.

The purification method of the present invention is carried out in a non-adsorption mode. The non-adsorption mode means that the antibody-containing aqueous solution is brought into contact with the inorganic compound containing silicon dioxide and aluminum oxide, and a non-adsorbed fraction is recovered without adsorbing the target antibody to the inorganic compound containing silicon dioxide and aluminum oxide. Specifically, the pH, the electrical conductivity, the buffer solution, or the antibody concentration of the antibody-containing aqueous solution, or the antibody loading amount per unit volume of the inorganic compound containing silicon dioxide and aluminum oxide, the temperature, or the contact time with the inorganic compound containing silicon dioxide and aluminum oxide, or the like is adjusted in advance, and then, the aqueous solution is brought into contact with the inorganic compound containing silicon dioxide and aluminum oxide, whereby an impurity is adsorbed to the inorganic compound containing silicon dioxide and aluminum oxide without adsorbing the target antibody to the inorganic compound containing silicon dioxide and aluminum oxide, and the antibody with a reduced impurity content can be recovered in the non-adsorbed fraction.

As one embodiment of the purification method of the present invention, a purification method comprising equilibrating the inorganic compound containing silicon dioxide and aluminum oxide with a buffer solution as a pretreatment is exemplified. The equilibration is performed by bringing a buffer solution adjusted to an appropriate pH and an appropriate concentration into contact with the inorganic compound containing silicon dioxide and aluminum oxide. For example, a method in which an appropriate amount of the buffer solution is allowed to pass through the inorganic compound containing silicon dioxide and aluminum oxide, or a method in which the inorganic compound containing silicon dioxide and aluminum is mixed with the buffer solution for an appropriate time is exemplified. Whether or not the inorganic compound is equilibrated can be confirmed by measuring the pH of the buffer solution after it is brought into contact with the inorganic compound. For example, when the pH of the buffer solution after it is brought into contact with the inorganic compound is the pH before the contact ± 0.1, 0.2, 0.3, 0.4, 0.5, 0.6, 0.7, 0.8, 0.9, or 1, the inorganic compound containing silicon dioxide and aluminum is equilibrated.

The pH of the antibody-containing aqueous solution to be brought into contact with the inorganic compound containing silicon dioxide and aluminum oxide, or the buffer solution to be used for equilibrating the inorganic compound containing silicon dioxide and aluminum oxide is preferably 4 to 9, more preferably 5 to 9, particularly preferably 6 to 9, and further more preferably 7.5 to 8.5. Further, as a salt constituting the antibody-containing aqueous solution or the buffer solution to be used for the equilibration, for example, a phosphate, a citrate, an acetate, a succinate, a maleate, a borate, Tris (base), HEPES, MES, PIPES, MOPS, TES, Tricine, or the like is exemplified. The concentration thereof is preferably 0.01 mol/L to 0.5 mol/L. Further, the salt can also be used in combination with, for example, 0.01 mol/L to 0.5 mol/L, preferably 0.01 mol/L to 0.5 mol/L of another salt such as sodium chloride, potassium chloride, calcium chloride, sodium citrate, sodium sulfate, ammonium sulfate, or the like. Further, the buffer solution component can also be used in combination with, for example, an amino acid such as glycine, alanine, arginine, serine, threonine, glutamic acid, aspartic acid, or histidine, a sugar such as glucose, sucrose, lactose, or sialic acid, or a derivative thereof, or the like.

The temperature of the antibody-containing aqueous solution to be brought into contact with the inorganic compound containing silicon dioxide and aluminum oxide, or the buffer solution to be used for equilibrating the inorganic compound containing silicon dioxide and aluminum oxide is preferably 4°C to 60°C, more preferably 10°C to 50°C, and particularly preferably 20°C to 40°C.

In the present invention, an antibody with a reduced impurity content can be obtained with a high recovery rate by recovering the non-adsorbed fraction of the inorganic compound containing silicon dioxide and aluminum oxide. Specifically, it is possible to obtain the antibody in which as the impurity content, the content of an antibody-derived polymer is preferably 5% or less, more preferably 2% or less, and particularly preferably 1% or less. Whether or not the impurity content is reduced can be confirmed by, for example, calculating the proportion of an impurity adsorbed to the inorganic compound containing silicon dioxide and aluminum oxide (impurity adsorption ratio). In particular, when the impurity is an antibody-derived polymer (HMDS), the HMDS adsorption ratio is preferably 10% or more, 20% or more, 30% or more, 40% or more, 50% or more, 70% or more, 80% or more, or 90% or more. It is possible to obtain the antibody with a recovery rate of preferably 35% or more, more preferably 45% or more, and further more preferably 60% or more.

In the present invention, in the measurement of the content of an antibody and an impurity, an analysis method generally used in the purification of an antibody can be applied. For example, the content of an antibody can be measured by an absorbance, an affinity HPLC method such as protein A, or the like, the content of a host cell protein can be measured by an ELISA (Enzyme-Linked Immunosorbent Assay) method, a Western blotting method, an electrochemiluminescence method, or the like, the content of an antibody-derived polymer or an antibody-derived degradation product can be measured by a gel filtration HPLC method, an ion exchange HPLC method, a polyacrylamide gel electrophoresis method, a light scattering method, an ultracentrifugal method, or the like, and a DNA can be measured by an analysis method such as a PicoGreen method, a Threshold method, or a QPCR method.

Further, the present invention relates to a method for producing an antibody comprising a purification method for obtaining an antibody with a reduced impurity content by separating an antibody from an impurity using an inorganic compound containing silicon dioxide and aluminum oxide.

In the production method of the present invention, the purification method using the inorganic compound containing silicon dioxide and aluminum oxide can be combined with another purification method. As the purification method to be combined with the purification method using the inorganic compound containing silicon dioxide and aluminum oxide, any purification method may be used as long as it is a method suitable for producing a pharmaceutical product, but examples thereof include chromatography, alcohol fractionation, removal of precipitates, salting out, buffer solution exchange, concentration, dilution, filtration, virus inactivation, virus removal, and the like. As for the purification method to be combined with the purification method using the inorganic compound containing silicon dioxide and aluminum oxide, multiple types and numbers may be combined. Further, these purification methods can be carried out whether before or after the purification method using the inorganic compound containing silicon dioxide and aluminum oxide.

As a support or a membrane to be used in the chromatography to be combined with the purification method using the inorganic compound containing silicon dioxide and aluminum oxide, an affinity support, an ion exchange support, an ion exchange membrane, a gel filtration support, a hydrophobic interaction support, a reverse phase support, a hydroxyapatite support, a fluoroapatite support, a sulfated cellulose support, a sulfated agarose support, a mixed mode support, and the like similar to the above are exemplified.

The chromatography to be combined with the purification method using the inorganic compound containing silicon dioxide and aluminum oxide is carried out in an adsorption mode or in a non-adsorption mode depending on the purpose. Examples of the chromatography include protein A chromatography, anion exchange chromatography, cation exchange chromatography, hydrophobic interaction chromatography, mixed mode chromatography, and activated carbon chromatography.

The adsorption mode in the chromatography means that the aqueous solution to be subjected to the chromatography is brought into contact with the support or the membrane to adsorb the target antibody to the support or the membrane, and thereafter washing is performed as needed, and then, the adsorbed fraction is recovered by eluting the antibody with a buffer solution in which the pH, the electrical conductivity, a buffer solution component, a salt concentration, an additive, or the like is changed. The non-adsorption mode in the chromatography means that the aqueous solution to be subjected to the chromatography is brought into contact with the support or the membrane to adsorb the target antibody, and the non-adsorbed fraction is recovered without adsorbing the target antibody to the support or the membrane.

As the method for producing an antibody of the present invention, for example, a method for producing an antibody, in which all types of chromatography to be combined with the purification method using the inorganic compound containing silicon dioxide and aluminum oxide are performed in a non-adsorption mode (all negative chromatography) is exemplified.

In the aqueous solution to be subjected to the chromatography to be combined with the purification method using the inorganic compound containing silicon dioxide and aluminum oxide or the buffer solution to be used for washing, preferred conditions are selected for each of the pH, the electrical conductivity, the buffer solution component, the salt concentration, the additive, and the like.

In the selection of the conditions of the chromatography, a difference in physicochemical property between the target antibody and a compound desired to be separated, for example, a difference in isoelectric point, electrical charge, hydrophobicity, molecular size, steric structure, or the like can be utilized. As an elution method in the adsorption mode, a one-step elution method in which the target antibody is eluted by allowing a buffer solution having a specific salt concentration or pH so as to decrease the affinity between the target antibody and the support to pass therethrough, a stepwise method in which the target antibody is eluted by changing the salt concentration or the pH in a stepwise manner, or a gradient method in which the target antibody is eluted by continuously changing the salt concentration or the pH is exemplified.

As a salt constituting the buffer solution, for example, a phosphate, a citrate, an acetate, a succinate, a maleate, a borate, Tris (base), HEPES, MES, PIPES, MOPS, TES, Tricine, or the like is exemplified. Further, the salt can also be used in combination with, for example, another salt such as sodium chloride, potassium chloride, calcium chloride, sodium citrate, sodium sulfate, ammonium sulfate, or the like. Further, the buffer solution component can also be used in combination with, for example, an amino acid such as glycine, alanine, arginine, serine, threonine, glutamic acid, aspartic acid, or histidine, a sugar such as glucose, sucrose, lactose, or sialic acid, or a derivative thereof, or the like.

According to the production method of the present invention, an antibody with a reduced impurity content can be obtained with a high recovery rate. Specifically, it is possible to obtain the antibody in which as the impurity content, the content of an antibody-derived polymer is preferably 5% or less, more preferably 2% or less, and particularly preferably 1% or less. It is possible to obtain the antibody with a recovery rate of preferably 35% or more, more preferably 45% or more, and further more preferably 60% or more.

Examples

Hereinafter, the present invention will be described in more detail with reference to Examples. However, the present invention is not limited to these Examples.

[Example 1] Examination of adsorption effect of activated clay or aluminum silicate on antibody polymer from culture supernatant

A CHO cell culture solution containing an IgG-type human monoclonal antibody (MabA) was clarified by microfiltration, and a culture supernatant adjusted to pH 7.0 with citric acid and trishydroxymethylaminomethane was prepared. The antibody concentration in the prepared culture supernatant was 4.63 mg/mL.

Subsequently, to 4 mL of this solution, any one of four types of activated clay and two types of aluminum silicate manufactured by Mizusawa Industrial Chemicals, Ltd. shown in Table 1 was added in an amount of 62, 123, or 247 mg, and mixed by inversion for about 16 hours. Among the adsorbents used, the properties of two types of aluminum silicate are shown in Table 2. The mixed liquid was centrifuged at 2900 g for 10 minutes, and filtered through a microfiltration membrane (Millex GV 0.22 $\mu$m, manufactured by Merck Millipore Ltd., Millex is a registered trademark) to remove each adsorbent.

The concentration of the antibody contained in the filtrate was measured by HPLC using a protein A affinity column, and the recovery rate was calculated. The recovery rate is defined as a value calculated according to the following calculation formula.

$$[\text{recovery rate (\%)}] = [\text{antibody concentration after treatment with adsorbent (mg/mL)}]$$
$$\div [\text{antibody concentration before treatment with adsorbent (mg/mL)}] \times 100$$

Further, the proportion of a polymer of the antibody (HMWS) in the filtrate was measured by size exclusion chromatography, and the HMWS adsorption ratio of each adsorbent was calculated. The HMWS adsorption ratio is defined as a value calculated according to the following calculation formula. In the formula, the content of HMWS in the culture supernatant is expressed as the ratio (%) of the peak area of HMWS to the total peak area of HPLC.

$$[\text{HMWS adsorption ratio (\%)}] = \{[\text{content of HMWS in culture supernatant before}$$
$$\text{treatment with adsorbent (\%)}] - [\text{content of HMWS in culture supernatant after}$$
$$\text{treatment with adsorbent (\%)}]\} \div [\text{content of HMWS in culture supernatant before}$$
$$\text{treatment with adsorbent (\%)}] \times 100$$

The performance of each adsorbent was evaluated based on the HMWS adsorption ratio and the recovery rate. The HMWS adsorption ratio and the recovery rate of each of various types of adsorbents are shown in Table 1. In Table 1, Galleon Earth, Galleon, and Neobead are registered trademarks.

[Table 1]

| Type of adsorbent | Name of adsorbent | Form | Specific surface area (m²/g) | Pore volume (mL/g) | Average pore diameter (nm) | Silica/alumina ratio | Addition amount (mg)*1 | HMWS adsorption ratio (%) | Recovery rate (%) |
|---|---|---|---|---|---|---|---|---|---|
| Activated clay | Galleon Earth NVZ | powder | 250 | 0.35 | 5.6 | 6.5 | 62 | 32.9 | 98.7 |
| | | | | | | | 123 | 70.5 | 94.7 |
| | | | | | | | 247 | 94.7 | 83.1 |
| Activated clay | Galleon Earth NV | powder | 250 | 0.4 | 6.4 | 7.5 | 62 | 35.4 | 98.9 |
| | | | | | | | 123 | 73.0 | 92.6 |
| | | | | | | | 247 | 97.5 | 75.4 |
| Activated clay | Galleon Earth V2R | powder | 305 | 0.48 | 6.3 | 8.3 | 62 | 38.6 | 98.7 |
| | | | | | | | 123 | 80.8 | 88.6 |
| | | | | | | | 247 | 98.1 | 67.9 |
| Activated clay | Galleon Earth V2 | powder | 290 | 0.46 | 6.3 | 9.2 | 62 | 36.4 | 98.8 |
| | | | | | | | 123 | 75.0 | 90.6 |
| | | | | | | | 247 | 97.4 | 73.7 |
| Aluminum silicate | Galleon Neutral D2-Y | powder | 530 | 0.3 | 2.3 | 4.0 | 62 | 18.0 | 99.0 |
| | | | | | | | 123 | 58.9 | 98.9 |
| | | | | | | | 247 | 75.5 | 98.4 |
| Aluminum silicate | Neobead SA | spherical shape | 550 | 0.36 | 2.6 | No data | 62 | 11.2 | 100.0 |
| | | | | | | | 123 | 23.4 | 100.2 |
| | | | | | | | 247 | 45.0 | 100.7 |

*1: The amount of the adsorbent added to 4 mL of the culture supernatant

As shown in Table 1, in any type of activated clay and aluminum silicate, the HMWS adsorption ratio increases in proportion to the addition amount of each adsorbent, and the effect of reducing the content of HMWS in the culture supernatant was observed. Under the condition that 247 mg of the adsorbent was added to 4 mL of the culture supernatant, the recovery rate was 67% or more and an HMWS adsorption ratio of 94% or more was exhibited in all four types of activated clay.

Further, in a case where the adsorbent was aluminum silicate, when Galleon Neutral D2-Y (Mizusawa Industrial Chemicals, Inc., Galleon is a registered trademark) was used, the recovery rate was 98% and the HMWS adsorption ratio was 76%. In addition, in a case where Neobead SA (Mizusawa Industrial Chemicals Co., Ltd., Neobead is a registered trademark) was used, the antibody recovery rate was 100% and the HMWS adsorption ratio was 45%.

From the above results, it was revealed that activated clay and aluminum silicate have an effect of highly selectively adsorbing HMWS from the culture supernatant.

[Example 2] Examination of correlation of adsorption effect on HMWS from culture supernatant with addition amount of aluminum silicate (Galleon Neutral D2-Y) (Mizusawa Industrial Chemicals, Inc., Galleon is a registered trademark)

With respect to Galleon Neutral D2-Y (Mizusawa Industrial Chemicals, Inc., Galleon is a registered trademark), which is one type of aluminum silicate, and in which the recovery rate of the antibody was high and the HMWS adsorption ratio was relatively high among the evaluated adsorbents, a correlation of the HMWS adsorption ratio and the recovery rate with the addition amount was evaluated.

A CHO cell culture solution containing an IgG1-type human monoclonal antibody (MabA) was clarified by microfiltration, and a culture supernatant adjusted to pH 7.6 with citric acid and trishydroxymethylaminomethane was prepared. The antibody concentration in the prepared culture supernatant was 6.15 mg/mL.

Subsequently, to 3 mL of this solution, Galleon Neutral D2-Y (Mizusawa Industrial Chemicals, Inc., Galleon is a registered trademark) was added in an amount of 70, 140, 280, 560, or 1120 mg, and mixed by inversion for about 16 hours. The mixed liquid was centrifuged at 2900 g for 10 minutes, and filtered through a microfiltration membrane (Millex GV 0.22 $\mu$m, manufactured by Merck Millipore Ltd., Millex is a registered trademark) to remove the adsorbent.

With respect to the filtrate, the HMWS adsorption ratio and the recovery rate were calculated by the method described in Example 1, and the optimal addition amount of the adsorbent was evaluated. The results are shown in Table 2.

[Table 2]

| Addition amount (mg)*1 | Adsorption ratio (%) | Recovery rate (%) |
|---|---|---|
| 70 | 20.9 | 98.6 |
| 140 | 45.2 | 98.3 |
| 280 | 63.1 | 97.5 |
| 560 | 88.6 | 103.0 |
| 1120 | 90.5 | 88.9 |
| *1: The amount of the adsorbent added to 3 mL of the culture supernatant | | |

As shown in Table 2, when the addition amount of Galleon Neutral D2-Y (Mizusawa Industrial Chemicals Co., Ltd., Galleon is a registered trademark) was doubled up to 560 mg, the HMWS adsorption ratio was improved by about 20%, and the recovery rate was maintained at almost 100%. On the other hand, under the condition that the addition amount was 1120 mg, as compared with the condition that the addition amount was 560 mg, the adsorption ratio was not almost improved, but the recovery rate decreased to 88.9%. From the above results, it was revealed that the optimal addition amount exhibiting a high adsorption ratio while maintaining the recovery rate is 560 mg, and when it is converted to the treatment amount (mg) of the antibody per unit adsorbent (1 mg), a treatment amount of 0.033 mg/mg is optimal.

[Example 3] Confirmation test of adsorption effect of aluminum silicate (Galleon Neutral D2-Y) (Mizusawa Industrial Chemicals, Inc., Galleon is a registered trademark) on HMWS from culture supernatants of various antibody types

The adsorption effect of aluminum silicate (Galleon Neutral D2-Y) (Mizusawa Industrial Chemicals, Inc., Galleon is a registered trademark) on HMWS derived from various antibodies was evaluated.

CHO cell culture solutions containing any of four types of antibodies shown in Table 3 were clarified by microfiltration, and culture supernatants adjusted to pH 6.0 with citric acid and trishydroxymethylaminomethane were prepared.

Subsequently, to 4 mL of each of the culture supernatants, Galleon Neutral D2-Y (Mizusawa Industrial Chemicals, Inc., Galleon is a registered trademark) was added so that the treatment amount (mg) of the antibody per unit adsorbent (mg) was 0.033 mg/mg (Table 3), and mixed by inversion for about 16 hours. The mixed liquid was centrifuged at 2900 g for 10 minutes, and filtered through a microfiltration membrane (Millex GV 0.22 μm, manufactured by Merck Millipore Ltd., Millex is a registered trademark) to remove the adsorbent.

With respect to each filtrate, the HMWS adsorption ratio and the recovery rate were calculated by the method described in Example 1. The results are shown in Table 3.

[Table 3]

| Antibody type | Subclass | Antibody concentration (mg/mL) | Addition amount of adsorbent to 4 mL of culture supernatant (mg) | Adsorption ratio (%) | Recovery rate (%) |
|---|---|---|---|---|---|
| MabA | IgG1 | 4.63 | 563 | 92.6 | 91.2 |
| MabB | IgG1 | 2.80 | 340 | 85.4 | 94.6 |
| MabC | IgG1 | 1.97 | 240 | 46.0 | 98.4 |
| MabD | IgG1 | 1.34 | 163 | 60.7 | 93.2 |
| MabE | IgG4 | 2.31 | 281 | 76.5 | 96.7 |

As shown in Table 3, although there was a difference in the HMWS adsorption ratio depending on the type of antibody, an HMWS adsorption ratio of 40% or more was exhibited while maintaining a recovery rate of 90% or more in any of the culture supernatants. From the above results, it was revealed that Galleon Neutral D2-Y (Mizusawa Industrial Chemicals Co., Ltd., Galleon is a registered trademark) has an effect of highly selectively adsorbing HMWS from the culture supernatant regardless of the type of antibody.

[Example 4] Examination of adsorption effect of zeolite on HMWS in culture supernatant

A CHO cell culture solution containing an IgG1-type human monoclonal antibody (MabA) was clarified by microfiltration, and a culture supernatant adjusted to pH 6.0 with citric acid and trishydroxymethylaminomethane was prepared. The antibody concentration in the prepared culture supernatant was 4.07 mg/mL.

Subsequently, to 4 mL of this solution, any of 38 types of zeolites manufactured by Tosoh Corporation shown in Table 4 was added in an amount of 124, 248, or 496 mg, and mixed by inversion for about 16 hours. The mixed liquid was centrifuged at 2900 g for 10 minutes, and filtered through a microfiltration membrane (Millex GV 0.22 μm, manufactured by Merck Millipore Ltd., Millex is a registered trademark) to remove the zeolite.

[Table 4]

| Name of zeolite | Crystal type | Cation | Silica/alumina ratio | Pore diameter (nm) | Specific surface area (m²/g) |
|---|---|---|---|---|---|
| A-3 4-8# | A type | potassium | 2 | 0.3 | ND |
| A-4 4-8# | A type | sodium | 2 | 0.4 | ND |
| A-5 4-8# | A type | calcium | 2 | 0.5 | ND |
| F-9 4-8# | X type | sodium | 2.5 | 0.9 | ND |
| 920NHA | β type | template | 18 | 0.65 | 580 |
| 930NHA | β type | template | 27 | 0.65 | 590 |
| 940NHA | β type | template | 40 | 0.65 | 580 |
| 931HOA | β type | proton | 28 | 0.65 | 510 |
| 940HOA | β type | proton | 40 | 0.65 | 530 |
| 941HOA | β type | proton | 40 | 0.65 | 520 |
| 960HOA | β type | proton | 100 | 0.65 | 560 |
| 980HOA | β type | proton | 500 | 0.65 | 500 |

(continued)

| Name of zeolite | Crystal type | Cation | Silica/alumina ratio | Pore diameter (nm) | Specific surface area ($m^2/g$) |
|---|---|---|---|---|---|
| 990HOA | β type | proton | 1500 | 0.65 | 470 |
| 820NHA | ZSM-5 type | ammonium | 23 | 0.58 | 510 |
| 840NHA | ZSM-5 type | ammonium | 40 | 0.58 | 330 |
| 822HOA | ZSM-5 type | proton | 24 | 0.58 | 330 |
| 840HOA | ZSM-5 type | proton | 40 | 0.58 | 330 |
| 890HOA | ZSM-5 type | proton | 1500 | 0.58 | 310 |
| 891HOA | ZSM-5 type | proton | 1500 | 0.58 | 310 |
| 720NHA | ferrierite type | ammonium | 18 | 0.48 | 260 |
| 770NAA | ferrierite type | sodium | 100 | 0.48 | 240 |
| 720KOA | ferrierite type | potassium | 18 | 0.48 | 170 |
| 722HOA | ferrierite type | proton | 18 | 0.48 | ND |
| 642NAA | mordenite type | sodium | 18 | 0.7 | ND |
| 620HOA | mordenite type | proton | 15 | 0.7 | 400 |
| 640HOA | mordenite type | proton | 18 | 0.7 | 380 |
| 660HOA | mordenite type | proton | 30 | 0.7 | 400 |
| 690HOA | mordenite type | proton | 240 | 0.7 | 450 |
| 500KOA | L type | potassium | 6.1 | 0.8 | 290 |
| 341NHA | Y type | ammonium | 7 | 0.9 | 700 |
| 320NAA | Y type | ammonium | 5.5 | 0.9 | 660 |
| 320HOA | Y type | sodium | 5.5 | 0.9 | 550 |
| 330HUA | Y type | proton | 6 | 0.9 | 550 |
| 331HSA | Y type | proton | 6 | 0.9 | 600 |
| 350HUA | Y type | proton | 10 | 0.9 | 650 |
| 360HUA | Y type | proton | 15 | 0.9 | 550 |
| 385HUA | Y type | proton | 100 | 0.9 | 600 |
| 390HUA | Y type | proton | 500 | 0.9 | 630 |

With respect to the obtained samples, the HMWS adsorption ratio and the recovery rate were calculated by the method described in Example 1, and the performance of each zeolite was evaluated. The HMWS adsorption ratio of each of various types of zeolites is shown in Fig. 1, and the recovery rate is shown in Fig. 2.

As shown in Fig. 1, 24 out of 38 types of zeolites had an improved HMWS adsorption ratio in proportion to the addition amount of the zeolite, and an effect of reducing the content of HMWS in the culture supernatant was observed. Further, among these, 930NHA, 960HOA, 980HOA, 990HOA, 660HOA, and 690HOA showed a recovery rate of 70% or more and an HMWS adsorption ratio of 50% or more when the zeolite was added in an amount of 248 mg or 496 mg to 4 mL of the culture supernatant, and revealed to have an effect of highly selectively adsorbing HMWS from the culture supernatant.

930NHA, 960HOA, 980HOA, and 990HOA belong to the β-type zeolite, and 660HOA and 690HOA belong to the mordenite-type zeolite, and it was suggested that zeolites having these crystal structures are suitable for selective adsorption of HMWS.

The elution patterns analyzed by size exclusion chromatography with respect to the sample treated with 660HOA are shown in Figs. 3(A) and 3(B). Fig. 3(A) shows the elution pattern before the treatment with the adsorbent, and Fig. 3(B) shows the elution pattern after the treatment with the adsorbent. As shown in Figs. 3(A) and 3(B), the peak area clearly became smaller after the treatment with the adsorbent, and also from this result, it was suggested that the zeolite has an adsorption effect on HMWS.

[Example 5] Examination of adsorption effect of zeolite (HSZ-660HOA) (Tosoh Corporation, HSZ is a registered trademark) on HMWS from culture supernatant

In order to evaluate the adsorption effect of a zeolite (HSZ-660HOA) (Tosoh Corporation, HSZ is a registered trademark) to HMWS derived from various antibodies, the following test was performed.

CHO cell culture solutions containing any of three types of antibodies shown in Table 5 were clarified by microfiltration, and culture supernatants were prepared. To 4 mL of each of the culture supernatants, HSZ-660HOA (Tosoh Corporation, HSZ is a registered trademark) was added in an amount shown in Table 5, and mixed by inversion for about 16 hours. The mixed liquid was centrifuged at 2900 g for 10 minutes, and filtered through a microfiltration membrane (Millex GV 0.22 $\mu$m, manufactured by Merck Millipore Ltd., Millex is a registered trademark) to remove the adsorbent.

With respect to the filtrate, the HMWS adsorption ratio and the recovery rate were calculated by the method described in Example 1. The results are shown in Table 5.

[Table 5]

| Antibody type | Subclass | Antibody concentration (mg/mL) | Treatment amount of antibody per unit adsorbent (mg/mg) | Addition amount of adsorbent to 4 mL of culture supernatant (mg) | Adsorption ratio (%) | Recovery rate (%) |
|---|---|---|---|---|---|---|
| MabA | IgG1 | 4.00 | 0.0375 | 427 | 46.3 | 92.3 |
| MabB | IgG1 | 5.60 | 0.075 | 299 | 77.0 | 86.6 |
| MabF | IgG1 | 2.53 | 0.0375 | 270 | 30.0 | 93.0 |

As shown in Table 5, although there was a difference in the adsorption ratio depending on the type of antibody, an HMWS adsorption ratio of 30% or more was exhibited while maintaining a recovery rate of 86% or more even when any of the culture supernatants was used. From the above results, it was revealed that the zeolite has an effect of highly selectively adsorbing HMWS from the culture supernatant regardless of the type of antibody.

[Example 6] Examination of adsorption effect of zeolite (HSZ-660HOA) (Tosoh Corporation, HSZ is a registered trademark) on HMWS in crudely purified sample

An adsorption effect of a zeolite (HSZ-660HOA) (Tosoh Corporation, HSZ is a registered trademark) on HMWS contained in a crudely purified sample obtained by purifying a culture supernatant containing MabA was evaluated. A culture supernatant containing MabA or MabD obtained in the same manner as in Example 1 was loaded onto MabSelect SuRe manufactured by GE Healthcare, Inc., followed by washing with a 10 mM Tris-hydrochloric acid buffer (pH 7.0). Thereafter, elution was performed with a 100 mM glycine-hydrochloric acid buffer (pH 3.2), followed by neutralization to pH 7.0 with trishydroxymethylaminomethane, whereby a crudely purified sample was prepared.

HSZ-660HOA (Tosoh Corporation, HSZ is a registered trademark) in an amount shown in Table 6 was weighed and equilibrated by the following method. In the equilibration, a 100 mM phosphate buffer (pH 7) was used.

Equilibration method: 40 mL of the buffer was added to HSZ-660HOA (Tosoh Corporation, HSZ is a registered trademark) and mixed by inversion for about 1 hour. The mixed liquid was centrifuged at 2000 g for 10 minutes, and the supernatant was removed. The addition and removal of the buffer were repeated until the pH of the removed supernatant became 7.

To the equilibrated zeolite, the crudely purified sample containing MabA or MabD was added in an amount of 4 mL each, and mixed by inversion for about 16 hours. The mixed liquid was centrifuged at 2000 g for 10 minutes, and the supernatant was filtered through a microfiltration membrane (Millex GV 0.22 $\mu$m, manufactured by Merck Millipore Ltd., Millex is a registered trademark) to remove the zeolite.

With respect to the filtrate, the HMWS adsorption ratio was calculated by the method described in Example 1. The results are shown in Table 6.

[Table 6]

| Antibody type | Subclass | Antibody concentration (mg/mL) | Treatment amount of antibody per unit adsorbent (mg/mg) | Addition amount of adsorbent to 4 mL of crudely purified sample (mg) | Adsorption ratio (%) | Recovery rate (%) |
|---|---|---|---|---|---|---|
| MabA | IgG1 | 18.39 | 0.0375 | 1997 | 37.3 | 54.1 |
| MabD | IgG1 | 10.60 | 0.0375 | 1254 | 61.6 | 59.5 |

Although there was a difference in the adsorption ratio depending on the type of antibody, an adsorption effect on HMWS was observed in any of the crudely purified samples. From the above results, it was revealed that the zeolite has an effect of adsorbing HMWS not only from a culture supernatant, but also from a crudely purified sample regardless of the type of antibody.

The elution patterns analyzed by size exclusion chromatography with respect to the crudely purified sample containing MabD before and after purification with 660HOA are shown in Figs. 4(A) and 4(B). Fig. 4(A) shows the elution pattern before the treatment with the adsorbent, and Fig. 4(B) shows the elution pattern after the treatment with the adsorbent. As shown in Figs. 4(A) and 4(B), the peak area of HMWS became smaller after the treatment with the adsorbent [peak area before reaction: 987430 ($\mu$V*sec), peak area after reaction: 388219 ($\mu$V*sec)], and also from this result, it was suggested that the zeolite has an adsorption effect on HMWS.

[Example 7] Examination of optimal pH of adsorption effect of zeolite (HSZ-660HOA) (Tosoh Corporation, HSZ is a registered trademark) on HMWS in crudely purified sample

An adsorption effect of a zeolite (HSZ-660HOA) (Tosoh Corporation, HSZ is a registered trademark) on HMWS contained in a crudely purified sample obtained by purifying a culture supernatant containing MabD was evaluated.

A crudely purified sample of MabD obtained in the same manner as in Example 6 was adjusted to pH 6, 7, or 8 with hydrochloric acid and trishydroxymethylaminomethane. HSZ-660HOA in an amount shown in Table 7 was weighed and equilibrated by the method shown in Example 6. In the equilibration, a 100 mM phosphate buffer (pH 6, 7, or 8) was used.

To the equilibrated zeolite, the crudely purified sample containing MabD after adjustment of the pH was added in an amount of 4 mL each, and mixed by inversion for about 16 hours. The mixed liquid was centrifuged at 2000 g for 10 minutes, and the supernatant was filtered through a microfiltration membrane (Millex GV 0.22 $\mu$m, manufactured by Merck Millipore Ltd., Millex is a registered trademark) to remove the zeolite.

With respect to the filtrate, the HMWS adsorption ratio was calculated by the method described in Example 1. The results are shown in Table 7.

[Table 7]

| pH of crudely purified sample and buffer solution for equilibration | Antibody concentration (mg/mL) | Treatment amount of antibody per unit adsorbent (mg/mg) | Addition amount of adsorbent to 4 mL of crudely purified sample (mg) | Adsorption ratio (%) | Recovery rate (%) |
|---|---|---|---|---|---|
| pH 6 | 11.02 | 0.0375 | 1254 | 51.4 | 38.4 |
| pH 7 | 11.19 | 0.0375 | 1254 | 60.0 | 40.5 |
| pH 8 | 11.20 | 0.0375 | 1254 | 68.9 | 42.6 |

As shown in Table 7, an adsorption effect on HMWS was observed in the crudely purified samples adjusted to any pH. Above all, the HMWS adsorption ratio was highest when the pH was 8. From the above results, it was revealed that the optimal pH for adsorbing a polymer by the zeolite is 8.

From the above results, it was demonstrated that an inorganic powder containing a silicon atom and an aluminum atom such as activated clay, aluminum silicate, or a zeolite can be utilized for the purification of an antibody in a non-adsorption mode.

While the present invention has been described in detail and with reference to specific embodiments, it is obvious to those skilled in the art that various changes and modifications can be made without departing from the spirit and scope of the present invention. The present application is based on a Japanese patent application filed on July 31, 2019 (Patent Application No. 2019-141768), which is incorporated by reference in its entirety.

**Claims**

1. A method for purifying an antibody, comprising separating an antibody from an impurity in a non-adsorption mode using an inorganic compound containing silicon dioxide and aluminum oxide, thereby obtaining an antibody with a reduced impurity content.

2. The purification method according to claim 1, wherein the antibody is a genetically recombinant antibody.

3. The purification method according to claim 1 or 2, wherein the impurity is at least one selected from a host cell protein, an antibody-derived polymer, an antibody-derived degradation product, and a DNA.

4. The purification method according to claim 1 or 2, wherein the impurity is an antibody-derived polymer.

5. The purification method according to any one of claims 1 to 4, wherein the inorganic compound containing silicon dioxide and aluminum oxide is activated clay, aluminum silicate, or a zeolite.

6. The purification method according to any one of claims 1 to 5, wherein the inorganic compound containing silicon dioxide and aluminum oxide is aluminum silicate.

7. The purification method according to claim 6, wherein the aluminum silicate has at least one property selected from the following (i) to (iv):

    (i) a specific surface area is 300 to 800 $m^2/g$;
    (ii) a pore volume is 0.1 to 0.5 mL/g;
    (iii) an average pore diameter is 1.5 to 4 nm; and
    (iv) a silica/alumina ratio is 1 to 5.

8. The purification method according to claim 6 or 7, wherein the aluminum silicate is Galleon Neutral D2-Y (Mizusawa Industrial Chemicals, Ltd., Galleon is a registered trademark) or Neobead SA (Mizusawa Industrial Chemicals, Ltd., Neobead is a registered trademark).

9. The purification method according to any one of claims 1 to 5, wherein the inorganic compound containing silicon dioxide and aluminum oxide is activated clay.

10. The purification method according to claim 9, wherein the activated clay has at least one property selected from the following (i) to (iv):

    (i) a specific surface area is 100 to 400 $m^2/g$;
    (ii) a pore volume is 0.2 to 0.6 mL/g;
    (iii) an average pore diameter is 3 to 10 nm; and
    (iv) a silica/alumina ratio is 6 to 10.

11. The purification method according to claim 9 or 10, wherein the aluminum silicate is Galleon Earth NVZ, Galleon Earth NV, Galleon Earth V2R, or Galleon Earth V2 (Mizusawa Industrial Chemicals, Ltd., Galleon is a registered trademark).

12. The purification method according to any one of claims 1 to 5, wherein the inorganic compound containing silicon dioxide and aluminum oxide is a zeolite.

13. The purification method according to claim 12, wherein the zeolite has at least one property selected from the following (i) to (iii):

    (i) a specific surface area is 100 to 800 $m^2/g$;

(ii) a pore diameter is 0.2 to 1 nm; and
(iii) a silica/alumina ratio is 2 to 1500.

14. The purification method according to claim 12 or 13, wherein the zeolite is a β-type or mordenite-type zeolite.

15. The purification method according to any one of claims 12 to 14, wherein the zeolite contains a proton as a counter cation.

16. The purification method according to any one of claims 1 to 15, wherein the separation of the antibody from the impurity is performed at pH 4 to 9.

17. The purification method according to any one of claims 1 to 15, wherein the separation of the antibody from the impurity is performed at pH 6 to 9.

18. The purification method according to any one of claims 1 to 15, wherein the separation of the antibody from the impurity is performed at pH 7.5 to 8.5.

19. The purification method according to any one of claims 1 to 18, which comprises equilibrating the inorganic compound containing silicon dioxide and aluminum oxide with a buffer solution as a pretreatment.

20. A method for producing an antibody, comprising the purification method according to any one of claims 1 to 19.

21. The production method according to claim 20, which comprises at least one of protein A chromatography, anion exchange chromatography, cation exchange chromatography, hydrophobic interaction chromatography, mixed mode chromatography, and activated carbon chromatography.

22. An antibody produced by the production method according to claim 20 or 21.

Fig. 1

Fig. 2

# Fig. 3

(A)

(B)

# Fig. 4

(A)

(B)

## INTERNATIONAL SEARCH REPORT

International application No.

PCT/JP2020/029150

### A. CLASSIFICATION OF SUBJECT MATTER
C07K 1/14(2006.01)i; C07K 16/00(2006.01)i; C12P 21/08(2006.01)i
FI: C07K1/14; C07K16/00; C12P21/08
According to International Patent Classification (IPC) or to both national classification and IPC

### B. FIELDS SEARCHED
Minimum documentation searched (classification system followed by classification symbols)
C07K1/14; C07K16/00; C12P21/08

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | JP 60-6622 A (FUJIREBIO INC.) 14.01.1985 (1985-01-14) claims, page 2, example 3 | 1, 5-6, 16-20, 22 |
| Y | claims, page 2, example 3 | 2-4, 7-8 |
| A | claims | 9-15, 21 |
| | | |
| Y | JP 2015-117272 A (HITACHI CHEMICAL CO., LTD.) 25.06.2015 (2015-06-25) claims, paragraph [0029] | 7-8 |
| A | claims | 1-6, 9-22 |
| | | |
| X | JP 57-95918 A (SAIKIN KAGAKU KENKYUSHO KK) 15.06.1982 (1982-06-15) claims, pp. 2, 4 | 1, 5, 9, 20-22 |
| Y | claims, pp. 2, 4 | 2-4, 10-11 |
| A | claims | 6-8, 12-19 |
| | | |
| Y | JP 2001-129392 A (TAKEDA PHARMACEUTICAL COMPANY LIMITED) 15.05.2001 (2001-05-15) claims, paragraph [0007] | 10-11 |
| A | claims | 1-9, 12-22 |

☒ Further documents are listed in the continuation of Box C.     ☒ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 September 2020 (01.09.2020) | 24 September 2020 (24.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.

PCT/JP2020/029150

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2017-226678 A (KYOWA HAKKO KIRIN CO., LTD.) 28.12.2017 (2017-12-28) claims, paragraphs [0003]-[0005] | 2-4 |
| A | claims | 1, 5-22 |

Form PCT/ISA/210 (continuation of second sheet) (January 2015)

| International application No. |
| --- |
| PCT/JP2020/029150 |

| Patent Documents referred in the Report | Publication Date | Patent Family | Publication Date |
| --- | --- | --- | --- |
| JP 60-6622 A | 14 Jan. 1985 | (Family: none) | |
| JP 2015-117272 A | 25 Jun. 2015 | (Family: none) | |
| JP 57-95918 A | 15 Jun. 1982 | (Family: none) | |
| JP 2001-129392 A | 15 May 2001 | (Family: none) | |
| JP 2017-226678 A | 28 Dec. 2017 | US 2014/0046038 A1 claims, paragraphs [0003]-[0005] EP 2883882 A1 | |

Form PCT/ISA/210 (patent family annex) (January 2015)

## REFERENCES CITED IN THE DESCRIPTION

### Patent documents cited in the description

- JP 2010528076 T
- WO 2007062245 A
- WO 2007114496 A
- JP 2019141768 A

### Non-patent literature cited in the description

- **A. A. SHUKLA et al.** *Journal of Chromatography B,* 2007, vol. 848, 28-39
- **M. MATSUI et al.** *Chemistry-European Journal,* 2001, vol. 7 (7), 1555-1560
- **Y. C. HUANG et al.** *Enzyme Microb. Technol.,* 1995, vol. 17, 564-569
- **Y. C. YU et al.** *Biotechnol. Prog.,* 1998, vol. 14, 332-337
- *The Journal of the American Medical Association,* 1967, vol. 199, 519
- *Science,* 1952, vol. 122, 501
- *Virology,* 1959, vol. 8, 396
- *Proceeding of the Society for the Biological Medicine,* 1950, vol. 73, 1
- *Proc. Natl. Acad. Sci. USA,* 1965, vol. 53, 288
- *J. Experimental Medicine,* 1978, vol. 147, 923